# EUROPEAN PATENT APPLICATION

(11) **EP 4 324 846 A1**
(43) Date of publication of application: **21.02.2024**
(21) Application number: 22190608.4
(22) Date of filing: 16.08.2022
(51) Int. Cl.: C07K 14/485, A61P 35/00, A61K 38/00

(54) **INHIBITOR PROTEIN OF LIGANDS OF EPIDERMAL GROWTH FACTOR RECEPTOR (EGFR)**

(71) Applicant: Eberhard Karls Universität Tübingen, Medizinische Fakultät, 72074 Tübingen (DE); Max-Planck-Institut für Medizinische Forschung, 69120 Heidelberg (DE)
(72) Inventor: Skokowa, Julia, 72127 Kusterdingen (DE); ElGamacy, Mohammad, 72076 Tübingen (DE); Maksymenko, Kateryna, 72076 Tübingen (DE)
(74) Representative: Witte, Weller & Partner Patentanwälte mbB

(57) **Abstract**

The present invention relates to an inhibitor protein of ligands of epidermal growth factor receptor (EGFR), an antibody or fragment thereof that specifically recognizes said inhibitor protein, a polynucleotide comprising a nucleotide sequence encoding said inhibitor protein or antibody or fragment thereof, a vector comprising said polynucleotide, a recombinant host cell comprising said inhibitor protein, polynucleotide or vector, a pharmaceutical composition comprising said inhibitor protein, the antibody or fragment thereof, the polynucleotide, the vector, or the recombinant host cell, a kit and a method of treating an EGFR-associated disease in a subject in need.

## Description

The present invention relates to an inhibitor protein of ligands of epidermal growth factor receptor (EGFR), an antibody or fragment thereof that specifically recognizes said inhibitor protein, a polynucleotide comprising a nucleotide sequence encoding said inhibitor protein or antibody or fragment thereof, a vector comprising said polynucleotide, a recombinant host cell comprising said inhibitor protein, polynucleotide or vector, a pharmaceutical composition comprising said inhibitor protein, the antibody or fragment thereof, the polynucleotide, the vector, or the recombinant host cell, a kit and a method of treating an EGFR-associated disease in a subject in need.

### FIELD OF THE INVENTION

The present invention relates to the field of molecular biology or medicine, more particular to the field of peptide active agents.

### BACKGROUND OF THE INVENTION

The epidermal growth factor receptor (EGFR) is a transmembrane protein that is a receptor for members of the epidermal growth factor family (EGF family) of extracellular protein ligands. Deficient signaling of the EGFR and other receptor tyrosine kinases in humans is associated with diseases such as Alzheimer's, while over-expression is associated with the development of a wide variety of tumors.

Especially, several cancer types have shown to be strongly dependent on EGFR signaling for survival, tumor growth progression and metastasis. This motivated the art to the develop different modes of EGFR inhibitors, specifically, small-molecule inhibitors of the receptor's intracellular kinase domain, or monoclonal antibodies blocking its ectodomain dimerization; see Chong, C.R. and P.A. Jänne, The quest to overcome resistance to EGFR-targeted therapies in cancer. Nature Medicine, 2013. 19(11): p. 1389-1400.

Such inhibitors have been in clinical use for treating different EGFR-dependent cancers, including colon cancer and epidermoid carcinomas; see Chan, D.L.H., et al., Epidermal growth factor receptor (EGFR) inhibitors for metastatic colorectal cancer. Cochrane Database of Systematic Reviews, 2017(6); Schrank, Z., et al., Current Molecular-Targeted Therapies in NSCLC and Their Mechanism of Resistance. Cancers, 2018. 10(7): p. 224.

These two inhibitors, i.e., tyrosine kinase inhibitors and dimerization-inhibiting monoclonal antibodies, however, have shown both to be subject to evasion by cancer cells through numerous evolution and resistance mechanisms; see Chong et al., 2013, *I.c.*

In the WO2009/052184 a multimeric protein-based inhibitor of EGFR ligands is disclosed. However, said inhibitor is characterized by its high molecular weight. Therefore, the production of the known inhibitor protein is quite costly and complex. It is typically expressed in mammalian cells but only with low efficiency.

It is, therefore, a problem underlying the invention, to provide a new compound capable of inhibiting the EGFR-mediated signal transduction, which can overcome the disadvantages of the current EGFR inhibitors or at least can significantly improve the problems associated therewith, making the new compound suitable as a therapeutic agent.

### SUMMARY OF THE INVENTION

This object is solved by the provision of an inhibitor protein of ligands of epidermal growth factor receptor (EGFR), or a pharmaceutically acceptable salt or solvate thereof, wherein the peptide inhibitor comprises the amino acid sequence of SEQ ID NO: 1 and variant sequences thereof which are at least 88% homologous to SEQ ID NO: 1, and wherein said variant binds to ligands of EGFR, wherein said peptide inhibitor is not a full-length polypeptide.

According to the invention an "inhibitor protein" refers to a protein having the amino acid sequence described herein, and which exerts an inhibitory activity on a ligand of EGFR. Preferably the inhibitor protein complexes the ligand and thereby renders the latter unable to bind to EGFR or only to bind with reduced affinity. Because of the inhibitory activity of the inhibitor protein the ligand is then unable to trigger an EGFR-mediated, intracellular signal transduction cascade or will at least only be able to trigger said cascade to a reduced extent.

The "epidermal growth factor receptor" (EGFR; ErbB-1; HER1 in humans) is a transmembrane protein that is a receptor for members of the epidermal growth factor family (EGF family) of extracellular protein ligands (Entrez: 1956 (human), 13649 (mouse)). In an embodiment of the invention the human variant of EGFR and/or ligands thereof are of preference.

"Ligands" of EGFR are molecules which can specifically bind to EGFR and activate the latter. Such ligands include epidermal growth factor (EGF), transforming growth factor α (TGFα), heparin binding-EGF-like growth factor (HB-EGF), amphiregulin (AR), betacellulin (BTC), epiregulin (EREG), and epigen (EPGN).

The term "pharmaceutically acceptable salt", as used herein, represents salts or zwitterionic forms of the peptides or compounds of the present invention which are water or oil-soluble or dispersible, which are suitable for treatment of diseases without undue toxicity, irritation, and allergic response; which are commensurate with a reasonable benefit/risk ratio, and which are effective for their intended use. The salts can be prepared during the final isolation and purification of the compounds or separately by reacting an amino group with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxye-thansulfonate (isethionate), lactate, maleate, mesitylenesulfonate, methanesulfonate, naphthylenesulfonate, nicotinate, 2-naphthalenesulfonate, oxalate, pamoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Also, amino groups in the compounds of the present invention can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. A pharmaceutically acceptable salt may suitably be a salt chosen, e.g., among acid addition salts and basic salts. Examples of acid addition salts include chloride salts, citrate salts and acetate salts. Examples of basic salts include salts where the cation is selected among alkali metal cations, such as sodium or potassium ions, alkaline earth metal cations, such as calcium or magnesium ions, as well as substituted ammonium ions, such as ions of the type N(R¹)(R²)(R³)(R⁴)+, where R¹, R², R³ and R⁴ independently will typically designate hydrogen, optionally substituted C₁₋₆-alkyl or optionally substituted C₂₋₆-alkenyl.

"Solvates" for the purposes of the invention refer to those forms of the inhibitor protein of the invention, which in the solid or liquid state form a complex by coordination with solvent molecules. Hydrates are a specific form of solvates in which the coordination takes place with water.

By a "variant" of the given amino acid sequence the inventors mean that the side chains of, for example, one or two or more of the amino acid residues are altered (for example by replacing them with the side chain of another naturally occurring amino acid residue or some other side chain) such that the protein is still able to inhibit the EGFR ligand in substantially the same way as a protein comprising the amino acid sequence of SEQ ID NO: 1. For example, a protein may be modified so that it at least maintains, if not improves, the ability to interact with and bind to the EGFR ligand.

The original (unmodified) inhibitor protein as disclosed herein can be modified by the substitution of one or more residues at different, possibly selective, sites within the peptide chain, if not otherwise stated. Preferably those substitutions are located at the end of the amino acid chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions". Conservative substitutions are herein defined as exchanges within one of the following five groups: Group 1-small aliphatic, nonpolar or slightly polar residues (Ala, Ser, Thr, Pro, Gly); Group 2-polar, negatively charged residues and their amides (Asp, Asn, Glu, Gln); Group 3-polar, positively charged residues (His, Arg, Lys); Group 4-large, aliphatic, nonpolar residues (Met, Leu, Ile, Val, Cys); and Group 5-large, aromatic residues (Phe, Tyr, Trp). Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such "radical" substitutions cannot, however, be dismissed as potentially ineffective since chemical effects are not totally predictable and radical substitutions might well give rise to serendipitous effects not otherwise predictable from simple chemical principles. Of course, such substitutions may involve structures other than the common L-amino acids. Thus, D-amino acids might be substituted for the L-amino acids commonly found in the antigenic peptides of the invention and yet still be encompassed by the disclosure herein. In addition, non-standard amino acids (i.e., other than the common naturally occurring proteinogenic amino acids) may also be used for substitution purposes to produce immunogens and immunogenic polypeptides according to the present invention.

If substitutions at more than one position are found to result in a peptide with substantially equivalent or greater inhibitory activity, then combinations of those substitutions will be tested to determine if the combined substitutions result in additive or synergistic effects on the inhibitory activity of the peptide.

The amino acid residues that do not substantially contribute to interactions with the EGFR ligands can be modified by replacement with other amino acids whose incorporation does not eliminate binding to the EGFR.

The amino acids of the inhibitor protein according to the invention include naturally occurring amino acids (e.g., a-amino acids), unnatural amino acids, modified amino acids, and non-natural amino acids. It includes both D- and L-amino acids. Unnatural or non-natural amino acids also include modified amino acids. "Modified" amino acids include amino acids (e.g., natural amino acids) that have been chemically modified to include a group, groups, or chemical moiety not naturally present on the amino acid.

In the present invention, the term "homologous" refers to the degree of identity between sequences of two amino acid sequences, i.e., peptide or polypeptide or protein sequences. The aforementioned "homology" is determined by comparing two sequences aligned under optimal conditions over the sequences to be compared. Such a sequence homology can be calculated by creating an alignment using, for example, the ClustalW algorithm. Commonly available sequence analysis software, more specifically, Vector NTI, GENETYX or other tools are provided by public databases.

"Percent identity/homology" or "percent identical/homologous" in turn, when referring to a sequence, means that a sequence is compared to a claimed or described sequence after alignment of the sequence to be compared (the "Compared Sequence") with the described or claimed sequence (the "Reference Sequence"). The percent identity is then determined according to the following formula: percent identity = 100 [1 -(C/R)]
wherein C is the number of differences between the Reference Sequence and the Compared Sequence over the length of alignment between the Reference Sequence and the Compared Sequence, wherein
(i) each base or amino acid in the Reference Sequence that does not have a corresponding aligned base or amino acid in the Compared Sequence and
(ii) each gap in the Reference Sequence and
(iii) each aligned base or amino acid in the Reference Sequence that is different from an aligned base or amino acid in the Compared Sequence, constitutes a difference and
(iv) the alignment has to start at position 1 of the aligned sequences;
and R is the number of bases or amino acids in the Reference Sequence over the length of the alignment with the Compared Sequence with any gap created in the Reference Sequence also being counted as a base or amino acid.

According to the invention "full-length polypeptide" refers to the source proteins from which the inhibitor protein is derived. In one embodiment of the invention "full-length polypeptides" or source proteins include human EGFR and/or the ligand binding domain thereof.

The inhibitor protein disclosed in accordance with the present invention may be in "purified" form. The term "purified" does not require absolute purity; rather, it is intended as a relative definition, and can include preparations that are highly purified or preparations that are only partially purified, as those terms are understood by those of skill in the relevant art. Purification of starting material or natural material to at least one order of magnitude, preferably two or three orders, and more preferably four or five orders of magnitude is expressly contemplated. Furthermore, a claimed inhibitor protein which has a purity of preferably 99.999%, or at least 99.99% or 99.9%; and even desirably 99% by weight or greater is expressly encompassed.

The inventors were able to identify said inhibitor protein via computational protein design and the use of one of the human EGFR ligand binding domains as starting template. The inventors were able to demonstrate that the inhibitor protein has significantly higher inhibitory and ligand binding activities compared to the natural EGFR ligand binding domains. Furthermore, it also showed good thermostability, comparable to that of the natural EGFR. Due to its small size, which can be as low as approximately 18 kDa, the lack of any posttranslational modifications, it is easily produced and purified as a recombinant protein, e.g., in common bacterial expression systems such as *E. coli.* The inhibitor protein is capable to bind and inhibit the family of EGFR ligands with broad specificity.

In contrast to the protein inhibitor known from WO2009/052184, the inventors adopt a strategy that aims at stabilizing the bound conformation of a single domain, using one of the EGFR ligand binding domains as starting template. As a result, the inventors have developed a small protein binder that can be expressed in *E. coli* and used as an inhibitor of EGFR ligands. To date, this represents the first report of a single-domain EGFR-mimic to inhibit its family of soluble ligands with broad specificity.

Biophysical experiments of the inventors demonstrated the inhibitor protein to bind EGF, TGF-α, and HB-EGF, which are growth factors involved in EGFR activation and cancer progression. *In vitro* assays carried out by the inventors showed ability of the inhibitor protein according to the invention to inhibit proliferation of the EGF-dependent epidermoid carcinoma cell line, A431. Quenching EGFR ligands by the inhibitor protein according to the invention can be implemented in combination with other already existing anti-cancer therapies, such as small-molecule inhibitors of EGFR intracellular kinase domain or monoclonal antibodies blocking EGFR ectodomain.

The object underlying the invention is herewith fully achieved.

In an embodiment of the invention the inhibitor protein comprises the following amino acid sequence: 
ANGIGIGEFKDSLSIX1AWNIKHFKNAX2SISGDLHILPVAFRGDSFTHX3PPLEPKELEILKT
VKEITGYLLX4QAWPENRTDLHAFENLEIIRGRTKQHGQFSLAVVSLNVTSLGLRSLKEISD
GDVIMSGNKNLKX5ANX6X7NWKX8VFGTSGQKTKIISNRGE (SEQ ID NO: 1),
 wherein:
X₁ = W or N
X₂ = R or Q
X₃ = T or M
X₄ = V or I
X₅ = M or W
X₆ = E or K
X₇ = M or W
X₈ = M or V.

With this measure, the inventors provide in an advantageous manner concretized amino acid sequences that result in inhibitor proteins that are characterized by particularly high inhibitory and binding activity.

It is understood that all sub-combinations of SEQ ID NO: 1 sequences are encompassed and disclosed according to the invention, i.e., each X₁ combined with each X₂, X₃, X₄, X₅, X₆, X₇, and X₈; each X₂ combined with each X₁, X₃, X₄, X₅, X₆, X₇, and X₈; each X₃ combined with each X₁, X₂, X₄, X₅, X₆, X₇, and X₈; each X₄ combined with each X₁, X₂, X₃, X₅, X₆, X₇, and X₈; each X₅ combined with each X₁, X₂, X₃, X₄, X₆, X₇, and X₈; each X₆ combined with each X₁, X₂, X₃, X₄, X₅, X₇, and X₈; each X₇ combined with each X₁, X₂, X₃, X₄, X₅, X₆, and X₈; each X₈ combined with X₁, X₂, X₃, X₄, X₅, X₆, and X₇.

In other embodiments of the invention the inhibitor proteins comprise the amino acid sequence of SEQ ID NO: 2 or SEQ ID NO: 3.

Inhibitor proteins with the amino acid sequence SEQ ID NO: 2 (internally also referred to as dd3-2) and the amino acid sequence SEQ ID NO: 3 (internally also referred to as dd3-1) were able to exert high ligand inhibitory and binding affinity, good thermostability and other properties as model inhibitors in model systems used by the inventors. They are therefore particularly suitable for therapeutic use.

Another subject-matter of the invention relates to an antibody, in particular a soluble or membrane-bound antibody, preferably a monoclonal antibody, or fragment thereof, that specifically recognizes the inhibitor protein according to the invention.

The term "antibody" or "antibodies" is used herein in a broad sense and includes both polyclonal and monoclonal antibodies. In addition to intact or "full" immunoglobulin molecules, also included in the term "antibodies" are fragments (e.g., CDRs, Fv, Fab and Fc fragments) or polymers of those immunoglobulin molecules and humanized versions of immunoglobulin molecules, as long as they exhibit any of the desired properties, i.e., specifically recognize the inhibitor protein according to the invention. Whenever possible, the antibodies of the invention may be purchased from commercial sources. The antibodies of the invention may also be generated using well-known methods.

The features, characteristics, advantages, and embodiments disclosed for inhibitor protein according to the invention apply to the antibody and fragment thereof correspondingly.

A still further subject-matter of the invention relates to a polynucleotide or nucleic acid molecule comprising a nucleotide sequence encoding the inhibitor protein and/or the antibody or fragment thereof according to the invention, optionally linked to a promoter sequence.

The polynucleotide or nucleic acid coding for said inhibitor protein or antibody or fragment thereof may be synthetically constructed or may be naturally occurring. The nucleic acid or polynucleotide may be, for example, DNA, cDNA, PNA, RNA or combinations thereof, either single- and/or double- stranded, or native or stabilized forms of polynucleotides, such as, for example, polynucleotides with a phosphorothioate backbone, and it may or may not contain introns so long as it codes for the protein. Of course, only peptides that contain naturally occurring amino acid residues joined by naturally occurring peptide bonds are encodable by a polynucleotide.

A still further aspect of the invention provides a vector, such as an expression vector, capable of expressing a/the protein(s) according to the invention.

As used herein the term "polynucleotide/nucleic acid coding for (or encoding) a peptide/protein" refers to a nucleotide sequence coding for the protein/peptide including artificial (man-made) start and stop codons compatible for the biological system the sequence is to be expressed. The term "promoter" means a region of DNA involved in the binding of the RNA polymerase to initiate transcription.

In an embodiment of the invention the polynucleotide or nucleic acid is isolated. The term "isolated" means that the material is removed from its original environment (e.g., the natural environment, if it is naturally occurring). For example, a naturally-occurring polynucleotide present in a living animal is not isolated, but the same polynucleotide, separated from some or all of the coexisting materials in the natural system, is isolated. Such polynucleotides could be part of a vector and/or such polynucleotides or (poly-) peptides could be part of a composition, and still be isolated in that such vector or composition is not part of its natural environment.

The features, characteristics, advantages and embodiments disclosed for the inhibitor protein according to the invention apply to the nucleic acid/polynucleotide and expression vector correspondingly.

Another subject-matter of the invention relates to a recombinant host cell comprising the inhibitor protein, and/or the polynucleotide, and/or the vector according to the invention.

The recombinant host cell includes a bacterial cell, such as *Escherichia coli,* or a non-bacterial cell, such as an insect or a mammal cell.

The features, characteristics, advantages and embodiments disclosed for the inhibitor protein according to the invention apply to the host cell correspondingly.

A yet other subject-matter of the invention relates to a pharmaceutical composition comprising the inhibitor protein, and/or the antibody or fragment thereof, and/or the polynucleotide, and/or the vector, and/or the recombinant host cell according to the invention.

A "pharmaceutical composition" is a composition suitable for administration to a human being in a medical setting. Preferably, a pharmaceutical composition is sterile and produced according to GMP guidelines.

The pharmaceutical composition comprises the inhibitor protein and/or the other active agents either in the free form or in the form of a pharmaceutically acceptable salt (see also above). It may be administered directly into the patient, into the affected organ or systemically *i.d.*, *i.m., s.c., i.p.* and *i.v.,* or applied ex *vivo* to cells derived from the patient or a human cell line which are subsequently administered to the patient.

The pharmaceutical composition according to the invention may comprise, as the only active ingredient, the inhibitor protein according to the invention. However, in an alternative aspect, it may comprise (an) additional active ingredient(s).

Another subject-matter of the invention relates to a kit comprising:
(a) a container comprising the inhibitor protein, and/or the antibody or fragment thereof, and/or the polynucleotide, and/or the vector, and/or the recombinant host cell according to the invention, in solution or in lyophilized formulation;
(b) optionally, a second container containing a diluent or reconstituting solution for the lyophilized formulation;
(c) optionally, instructions for (i) use of the solution or (ii) reconstitution and/or use of the lyophilized formulation.

The kit may further comprise one or more of (d) a buffer, (e) a diluent, (f) a filter, (g) a needle, or (h) a syringe. The container is preferably a bottle, a vial, a syringe or test tube; and it may be a multi-use container. The content of the first container is preferably lyophilized.

The kits of the present invention preferably comprise a lyophilized formulation of the present invention in a suitable container and instructions for its reconstitution and/or use. Suitable containers include, for example, bottles, vials (e.g. dual chamber vials), syringes (such as dual chamber syringes) and test tubes. The container may be formed from a variety of materials such as glass or plastic. Preferably the kit and/or container contain/s instructions on or associated with the container that indicates directions for reconstitution and/or use. For example, the label may indicate that the lyophilized formulation is to be reconstituted to peptide concentrations as described above. The label may further indicate which kind of administration the formulation is useful or intended for.

The features, characteristics, advantages and embodiments disclosed for the inhibitor protein according to the invention apply to the kit correspondingly.

Still, another subject-matter of the invention relates to a method of treating an EGFR-associated disease in a subject in need, comprising administering to the subject an effective amount of the inhibitor protein, and/or the antibody or fragment thereof, and/or the polynucleotide, and/or the vector, and/or the recombinant host cell, or the pharmaceutical composition according to the invention.

According to the invention, an "EGFR-associated disease" refers to a disorder or illness that is caused by a dysfunctional EGFR. This includes cancer, inflammatory diseases, such as psoriasis, eczema and atherosclerosis, wound healing and fibrosis.

In an embodiment of the invention the EGFR-associated disease is cancer, preferably said cancer is selected from the group consisting of: colon cancer, epidermoid cancer, adenocarcinoma, anal cancer, glioblastoma and epithelial cancer.

Mutations that lead to EGFR overexpression (known as upregulation or amplification) have been associated with a number of cancers, including adenocarcinoma of the lung (40% of cases), anal cancers, glioblastoma (50%) and epithelian tumors of the head and neck (80-100%). These somatic mutations involving EGFR lead to its constant activation, which produces uncontrolled cell division. In glioblastoma a specific mutation of EGFR, called EGFRvlll, is often observed. Mutations, amplifications or misregulations of EGFR or family members are implicated in about 30% of all epithelial cancers.

In all these cases of EGFR-associated cancer or other EGFR-associated diseases, the invention provides a beneficial remedy.

The "subject in need" may be any living being, preferably a mammal, further preferably a human being.

In an embodiment of the method according to the invention the inhibitor protein, and/or the polynucleotide, and/or the vector, and/or the recombinant host cell, and/or the pharmaceutical composition are administered to the subject by an oral, parenteral, intravenous, peritoneal, intradermal, subcutaneous, intramuscular, intrathecal, inhalation, vaporization, nebulization, sublingual, buccal, parenteral, rectal, intraocular, inhalation, topically, vaginal, or topical route of administration.

With this measure, the administration of the active agent according to the invention is carried out in a manner adapted to the circumstances and ensures the right way of bringing the agent into the patient.

The features, characteristics, advantages and embodiments disclosed for the inhibitor peptide according to the invention apply to the afore-referenced method correspondingly.

### EMBODIMENTS

The invention is now further explained by means of embodiments resulting in additional features, characteristics, and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention.

The invention is now further described and explained in further detail by referring to the following non-limiting examples and figures:

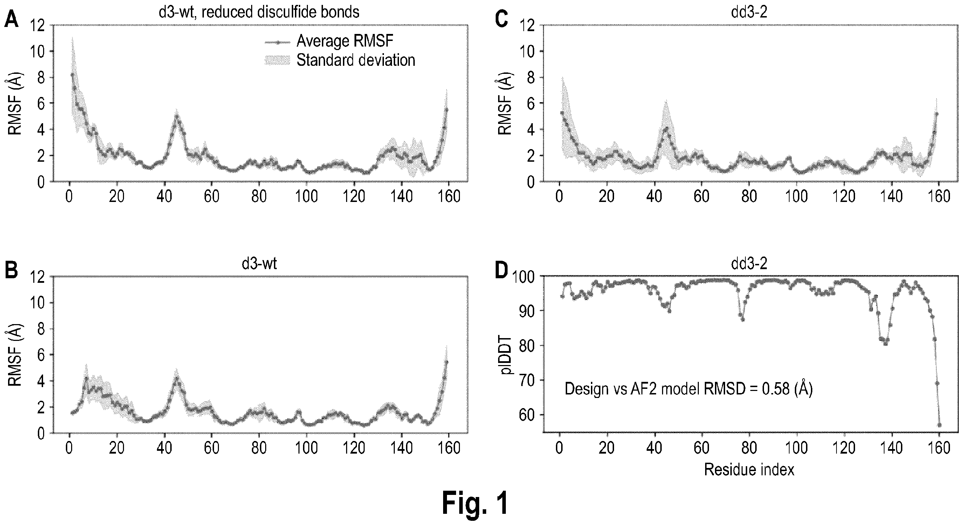

- Fig. 1:: Predicted conformational stability of designed and wild type d3. (A, B) RMSF plots of d3-wt with and without the N-terminal disulfide bridge strongly affects the domain's global and local stability. (C) RMSF plot of dd3-2 indicates improved stability compared to the reduced cysteines form of d3-wt. (D) Al-phaFold2 [43] model of dd3-2 shows strong prediction confidence across the sequence length, where the predicted structure had a backbone RMSD of 0.6 Å away from the design.

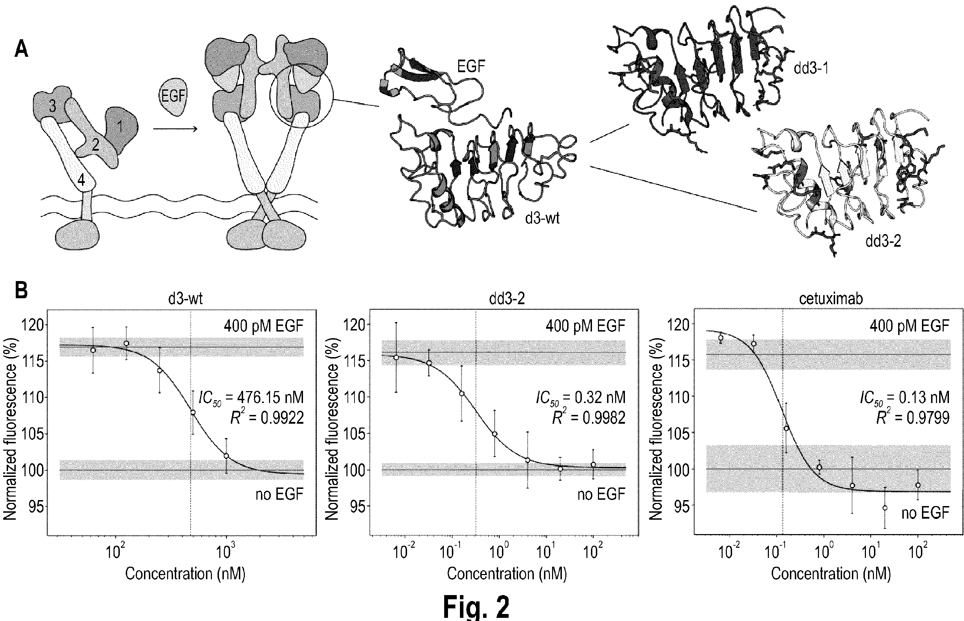

- Fig. 2:: The design and characterization of EGF inhibitors. (A) The EGFR extracellular segment consists of four domains (d1, violet; d2, cyan; d3 teal; and d4, yellow) that in absence of a ligand lies in a closed monomeric configuration. Upon lig- and binding (here EGF, gray), the receptor adopts an open, dimeric configuration capable of intracellular signaling. As the 3^{rd} domain of the EGFR is reported to hold most of the binding affinity to the EGF ligand, it was used as a template to design soluble EGF binders. A close up view of the EGF:d3 complex is shown in gray and teal colors, respectively (PDB: 1IVO). Using the Damietta scoring function, the highest energy residues were identified. These residues were defined as mutable (show in red) and designed using the Damietta f2m2f combinatorial sampler. Two design models (dd3-1; purple, and dd3-2; yellow) were finally chosen for experimental characterization. (B) Proliferation inhibition assays were done using the EGF signaling-dependent (A431) cells. The inhibition of cell proliferation was observed to be much stronger for dd3-2 (ICso = 0.320 nM) than for d3-wt (ICso = 476 nM), in comparison to a value of *IC₅₀* = 0.13 nM for the EGFR-blocking antibody Cetuximab. The positive and negative control values of cell proliferation with and without EGF-treatment are indicated by red and blue lines, respectively. Shades and error bars represent the standard deviation across three replicates.

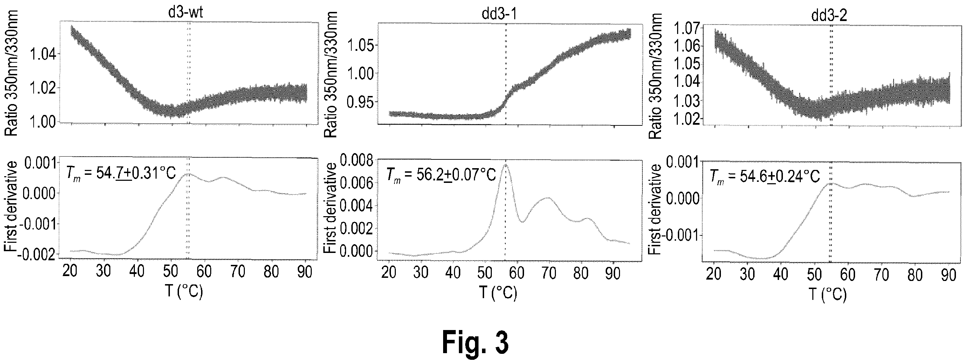

- Fig. 3:: NanoDSF melting curves. NanoDSF measurements showed no significant difference in melting temperatures between designed proteins (dd3-1, dd3-2) and wild type (d3-wt).

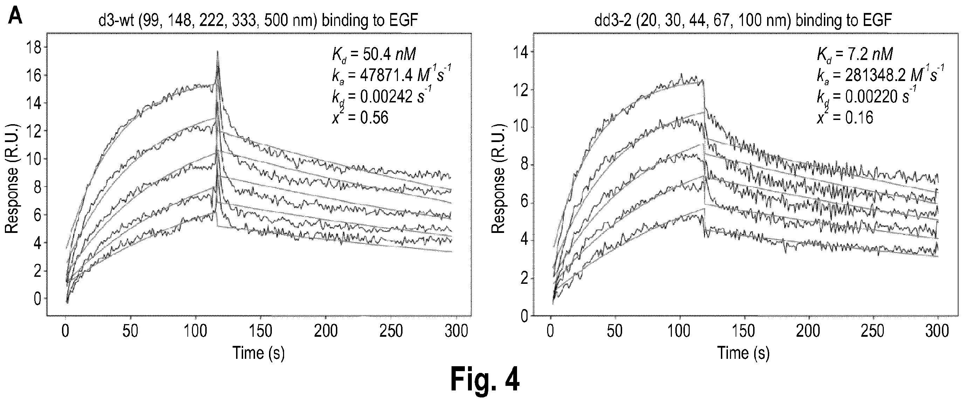

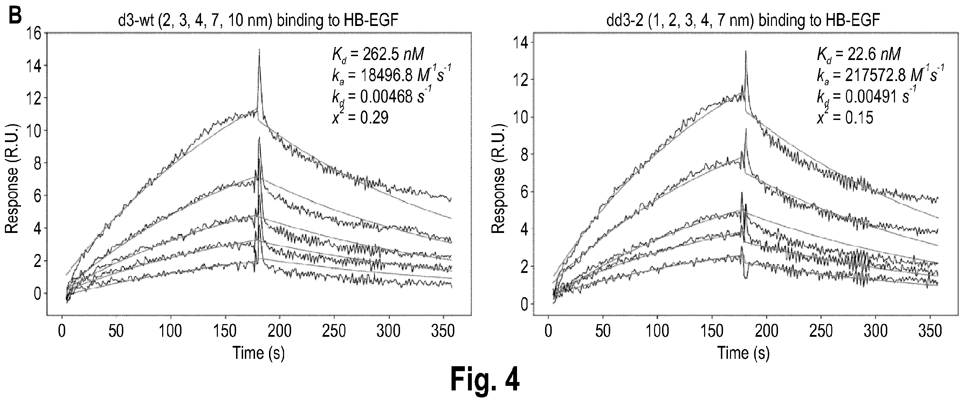

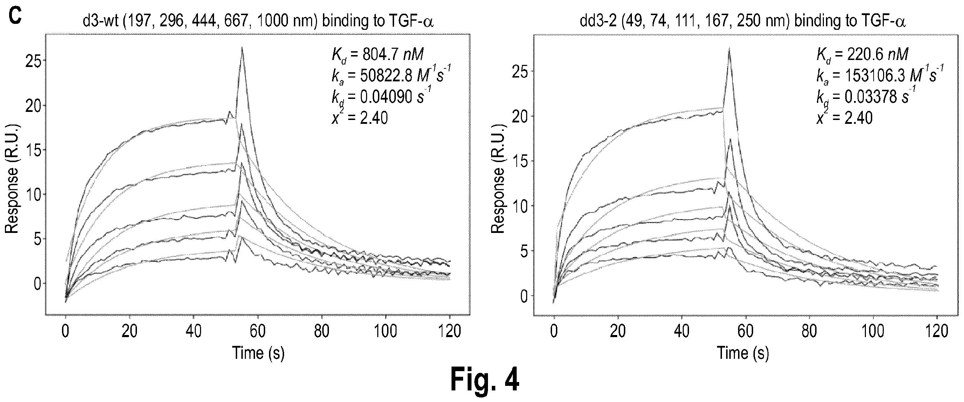

- Fig. 4:: The designed proteins bind to different EGFR ligands with high affinity. SPR sensograms of dd3-2 and d3-wt (A) show the dd3-2 design to bind EGF approximately 7-fold tighter than wild type d3 domain, with Kd values of 7 nM and 50 nM for design and wild type, respectively. (B) The same pattern was observed whereby an 11-fold affinity improvement was observed in HB-EGF binding, with Kd values of 23 nM and 263 nM for design and wild type, respectively. (C) Both design and wild type also bind TGF-α with Kd values of 805 nM and 221 nM, respectively. This highlights the broad spectrum of activity for a designed d3 domain.

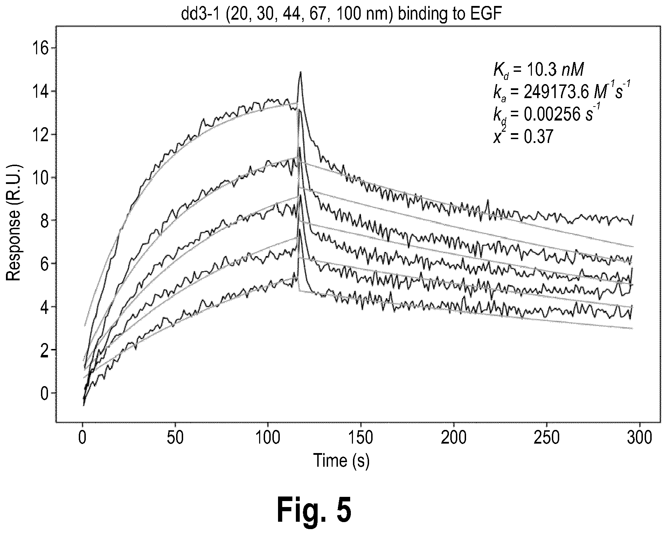

- Fig. 5:: SPR sensograms for dd3-1 binding to EGF. dd3-1 design was found to bind EGF approximately 5-fold tighter than wild-type d3 domain, with Kd values of 10 nM and 50 nM for design and wild-type, respectively.

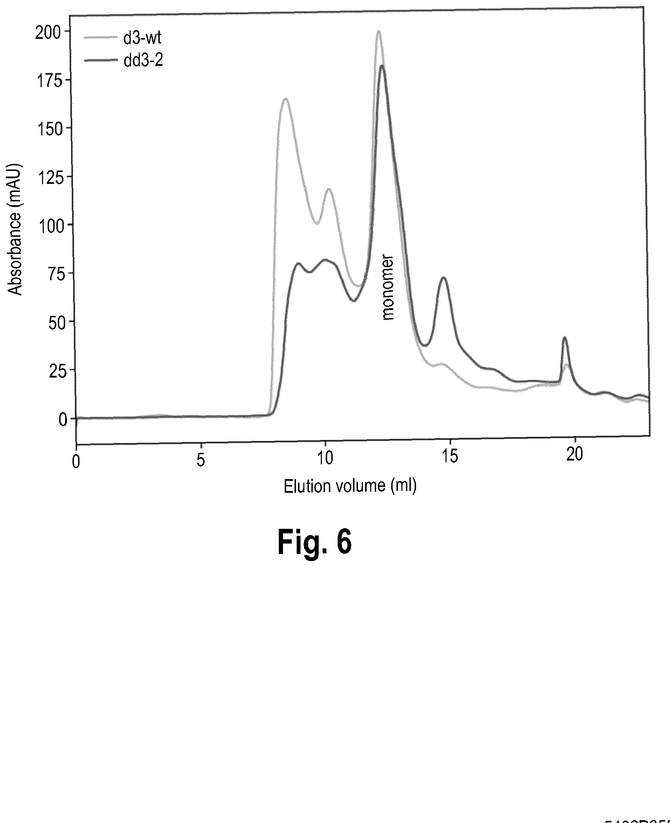

- Fig. 6:: Analytical size-exclusion elution profile of designed (red) and wild type (blue) d3. The chromatograms show d3-wt to have higher aggregation propensity than dd3-2. For experimental evaluation, monomeric fractions of dd3-2 and d3-wt were collected.

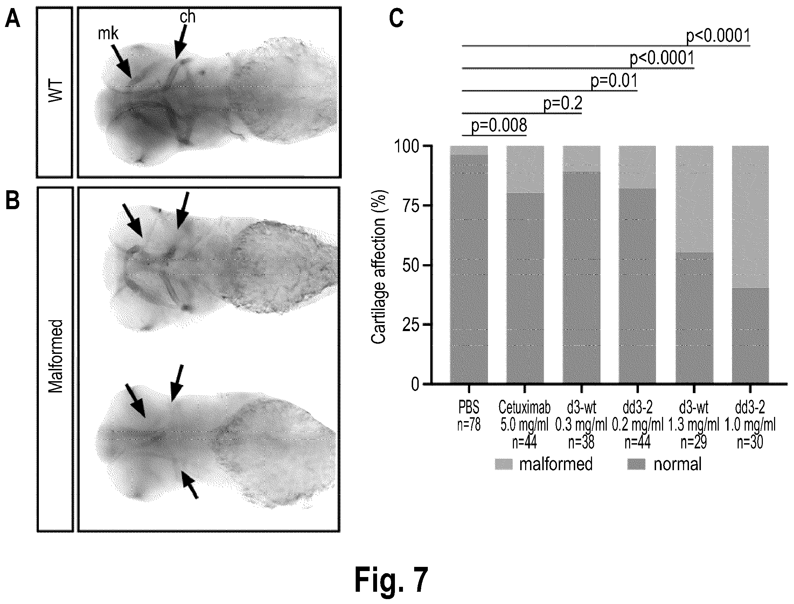

- Fig. 7:: Cartilage defect upon exposure to EGFR inhibitors. (A) Pharyngeal skeleton of zebrafish wild type (WT) embryos stained with Alcian blue. First arch Meckel's cartilage and second arch derivatives ceratobranchials are observable (arrows). (B) Upon EGFR pathway disruption embryos with partially absence of Meckel's cartilage and ceratobranchials (upper image) or without any cartilage formation (lower image) are categorized in malformed class. (C) Cartilage defection in percentages upon injection of PBS, Cetuximab, dd3-2, and d3-wt. Meckel's cartilage (mk), ceratobranchials (ch).

### 1. In silico protein design

The inventors sought to create a receptor-inspired, soluble quencher of the EGFR ligands, where the inhibition of EGFR signaling can serve as a therapeutic modality for several types of cancer. In this approach, they aim to design the receptor fragment that encodes most of the affinity to the ligand. In doing so, the inventors have created an exemplary non-antibody binder targeting EGF, as well as related EGFR ligands, that is as small as 18 kDa, devoid of any post-translational modifications, readily expressed and purified. To date, this represents the first report of a single-domain EGFR-mimic to inhibit its family of soluble ligands with broad specificity.

### 2. Material and methods

### Design of EGFR-based EGF binders

Computational design was performed using Damietta, a novel protein design framework. EGF:d3 structure (PDB: 1IVO) was used as a template (residue range: 313-480 for d3-wt, and 313-472 for designed proteins). Using the repack-all application (damietta_ra), the inventors had identified residues with energy higher than 20 kcal/mol, as well as all cysteine residues. These residues were subject to combinatorial design using the few-to-many-to-few sampler (damietta_cs_f2m2f). The target mutations in the spec file were specified according to a sequence profile of d3 homologues obtained from closest 500 homologous sequences in the nr protein database. The resulting decoys were further filtered according to their stability in accelerated molecular dynamics (AMD) simulations that follow a serial tempering routine previously described in the art. The two most conformationally homogeneous (quantified as the average all-vs.-all RMSD averaged across all frames output from the AMD simulations) designs (dd3-1 and dd3-2) in AMD simulations were accordingly selected for experimental characterization.

### Bacterial protein expression and purification of EGF binders

Sequence of d3-wt, and sequence of the designed proteins (dd3-1, dd3-2) were ordered as synthetic genes in pET-28a(+) expression vector (Synbio Technologies, Inc.). Plasmids were transformed into chemically competent *E. coli* BL21(DE3) using the heat shock method. Transformed cells were grown in LB medium supplemented with 40 µg/ml kanamycin at 37 °C. At OD600 of 0.6-1.0, cells were induced with 1mM IPTG and incubated overnight at 25°C for protein expression. Cells were harvested by centrifugation at 5000 g at 4°C for 20 min and lysed in 30 ml of lysis buffer (1M guanidinium chloride, 100 mM NaCl, 50mM Tris-HCI pH 8.0) supplemented with a tablet of the cOmplete, EDTA-free Protease Inhibitor Cocktail (Roche, 5056489001) and 3 mg of lyophilized DNase I (PanReac AppliChem, A3778) using a Branson Sonifier 250 (Fisher Scientific). The lysate was cleared by centrifugation at 28000 g at 4°C for 50 min and the supernatant was filtered through a 0.45 µm filter (Millipore, SLHV033RS). The sample was applied to a 5 ml HisTrap HP column (Cytiva, GE17-5248-01). The running buffer was 150 mM NaCl, 30 mM Tris-HCI pH 8.0. After sequential washing the column with 20 ml of the running buffer and 20 ml of the running buffer supplemented with 50 mM imidazole, fractions were collected by linear gradient elution using 150 mM NaCl, 30 mM Tris-HCI pH 8.0, 500 mM imidazole buffer. The eluted fractions containing the protein of interest were pooled, concentrated using 10 kDa MWCO centrifugal filters (Millipore, UFC901024), and further purified on a HiLoad 16/600 Superdex 200 gel filtration column (Cytiva, GE28-9893-35) using PBS. Gel filtration fractions containing pure protein in the desired oligomeric state were pooled, concentrated, and stored at -20 °C for subsequent analyses. Both IMAC and gel filtration steps were performed on an Äkta Pure chromatography system (Cytiva).

### Thermostability analysis of EGF binders

Nanoscale differential scanning fluorimetry (nanoDSF) using Prometheus NT.48 (Nanotemper) was applied to evaluate thermostability of the designs dd3-1, dd3-2, as well as thermostability of d3-wt. Capillaries (Nanotemper, PR-C002) were filled with 1-2 mg/ml protein samples in three replicates. Melting scan was performed across the temperature range from 20 °C to 90 °C with a temperature ramp of 1 °C/min.

### Surface plasmon resonance binding assays

Multi-cycle kinetics experiments were performed on a Biacore X100 system (GE Healthcare Life Sciences). For measuring binding to EGF, EGF (Peprotech AF-100-15) was diluted to 100 µg/mL in 10 mM acetate buffer pH 4.0 and immobilized on the surface of a CM5 sensor chip (GE Healthcare 22054642-AD) using standard amine coupling chemistry. Five sequential 1.5-fold increasing concentrations of the sample solution (for d3-wt from 99 nM to 500 nM; for dd3-1 and dd3-2 from 20 nM to 100 nM ) were injected over the functionalized sensor chip surface for 120 s, followed by a 180 s dissociation with running buffer. At the end of each run, the sensor surface was regenerated with a 30 s injection of 50 mM HCI at a flow rate of 10 µL/min. For measuring binding to HB-EGF, HB-EGF (R&D Systems 259-HE-050/CF) was diluted to 20 µg/mL in 10 mM acetate buffer pH 5.0 and immobilized on the surface of a CM5 sensor chip using standard amine coupling chemistry. Five sequential 1.5-fold increasing concentrations of the sample solution (for d3-wt from 2 nM to 10 nM; for dd3-2 from 1 nM to 7 nM ) were injected over the functionalized sensor chip surface for 180 s, followed by a 180 s dissociation with running buffer. At the end of each run, the sensor surface was regenerated with a 30 s injection of 50 mM NaOH at a flow rate of 10 µL/min. For measuring binding to TGF-α, TGF-α (R&D Systems 239-A-100) was diluted to 100 µg/mL in 10 mM acetate buffer pH 4.5 and immobilized on the surface of a CM5 sensor chip using standard amine coupling chemistry. Five sequential 1.5-fold increasing concentrations of the sample solution (for d3-wt from 197 nM to 1000 nM; for dd3-2 from 49 nM to 250 nM) were injected over the functionalized sensor chip surface for 60 s, followed by a 60 s dissociation with running buffer. At the end of each run, the sensor surface was regenerated with a 60 s injection of 10 mM glycine-HCI pH 1.5 at a flow rate of 10 µL/min. In all experiments, reference surfaces were treated in the same manner (surface activation and deactivation with amine coupling reagents), except that no ligand was added. Test proteins were diluted in running buffer (PBS supplemented with 0.05% v/v Tween-20). Analyses were conducted at 25°C at a flow rate of 10 µL/min. The reference responses and zero-concentration sensograms were subtracted from each dataset (double-referencing). Association rate (ka), dissociation rate (kd), and equilibrium dissociation (Kd) constants were obtained using Biacore Evaluation Software.

### A431 cell proliferation assay

A431 cells were cultured in DMEM medium (Gibco, 41966029) supplemented with 10 % FBS (Gibco, 10082147). Cells were pelleted by centrifugation at 300 g for 5 min, washed once with DPBS (Gibco, 14190144) and once with non-supplemented DMEM medium. After the last washing step, cells were resuspended in DMEM medium supplemented with 1 % FBS and 400 pM EGF (Peprotech AF-100-15). 100 µl of cell suspension were seeded in a 96-well plate (Corning, 3596) at a density of 8000 cells/well. Different concentrations of d3-wt (0.032 nM - 500 nM), dd3-2 (0.0064 nM - 100 nM), or cetuximab (0.0064 nM - 100 nM) were added to the wells in triplicates. PBS was added to the wells serving as an untreated control. Several wells contained cells in DMEM medium supplemented with 1 % FBS, but without EGF, as an unstimulated control. After incubation for 72 h at 37°C, 5% CO₂, 20 µL of CellTiter-Blue^{®} Reagent (Promega, G8080) were added to the wells and the plate was incubated for additional 2 h under the same conditions to allow cells to convert resazurin to resorufin. Cell viability was monitored by measuring fluorescence (560/590 nm) using a Synergy HTX Microplate Reader (BioTek). The data were presented as percentage of unstimulated (i.e., without EGF) control fluorescence values.

### Effect of the designed EGF binders on zebrafish embryos

Zebrafish lines were maintained according to standard protocols and handled in accordance with European Union animal protection directive 2010/63/EU and approved by the local government (Tierschutzgesetz §11, Abs. 1, Nr. 1, husbandry permit 35/9185.46/Uni TU). Eggs were collected and placed at 28 °C in E3 medium (5 mM NaCl, 0.17 mM KCI, 0.4 mM CaCl₂, and 0.16 mM MgSO₄). The age of the embryos and larvae is indicated as hours post fertilization (hpf) or days post fertilization (dpf). All experiments described in the present study were conducted on embryos younger than 5 days post-fertilization (dpf). To test the effect of the designed inhibitors on zebrafish, the inventors injected Cetuximab (5 mg/ml), dd3-2 (0.2 and 1 mg/ml), d3-wt (0.3 and 1.3 mg/ml) into the yolk of embryos at 4-6 hpf and then continued the treatment by adding the inhibitors into medium until 4 dpf. Embryos were distributed in pools of 10-15 into 24 well plates in E3 medium. Survival ratio was assessed every day from 1 dpf to 4 dpf and morphological or developmental defects were analyzed on fixed and stained embryos with Alcian blue at 4 dpf using a Nikon SMZ18 stereomicroscope with a DS-Fi3 camera (5,9 MP). GraphPad Prism software (version 7) was used for graphing and statistical analysis. Cartilage was stained with Alcian Blue 8 GX (Sigma). Zebrafish larvae were fixed in PFA 4 % for 2 h at room temperature, rinsed with PBST and stained overnight with 10 mM MgCl₂/80 % ethanol/0.04 % Alcian Blue solution. Embryos were rinsed with 80 % ethanol/10 mM MgCl₂ and washed stepwise with 70 %, 50 %, 30 % ethanol and PBST. Pigments were bleached in H₂O₂ 3 %/formamide 5 %/20X SSC 2,5 % up to 30 minutes. Embryos were stored in 80 % glycerol for imaging.

### Data accessibility

A pre-release of the Damietta software is available at https://bio.mpg.de/damietta.

### 3. Results

### Design and characterization of EGFR inhibitors

EGFR (HER1) represents an important target for modulating signal transduction cascades, as it dimerizes upon ligand-induced conformational change. Several cancer types have shown to be strongly dependent on EGFR signaling for survival, tumor growth progression and metastasis. This motivated the development of different modes of EGFR inhibitors, specifically, small-molecule inhibitors of the receptor's intracellular kinase domain, or monoclonal antibodies blocking its ectodomain dimerization. Such inhibitors have been in clinical use for treating different EGFR-dependent cancers, including colon cancer and epidermoid carcinomas. These two inhibition modalities (i.e., tyrosine kinase inhibitors and dimerization-inhibiting monoclonal antibodies) however have shown both to be subject to evasion by cancer cells through numerous evolution and resistance mechanisms. Therefore, the inventors decided to apply alternative approaches of developing EGFR inhibitors, that can be implemented in combination anti-cancer therapies for potential synergy. One such appealing approach is the direct inhibition of the soluble ligand itself - i.e. EGF. Adding to the complexion is the cross-activity of more than one EGF-family ligand against the EGFR and its related receptors, particularly the HER4 receptor. For instance, the heparin binding-EGF-like growth factor (HB-EGF), transforming growth factor-a (TGF-α), and amphiregulin (AR) do also play a role in activating these receptors, and promoting cancer progression, constituting additional targets for EGFR inhibitor design.

This multi-ligand nature of the EGFR motivates the development of poly-specific binders capable of quenching more than one growth factor. Previous efforts along this track have led to the engineering of a recombinant form of the entire extracellular segment of the EGFR and HER3 receptors to achieve broad ligand binding specificity. These binders were constructed as dimeric IgG1 Fc-fragment fusions with the 4 extracellular domains. While achieving broad ligand inhibition, these constructs require recombinant expression in mammalian cells, and possess large molecular weight of approximately 190 kDa, which is expected to hamper their bioavailability at the relevant tumor tissue. The inventors adopt a strategy that aims at stabilizing the bound conformation of a single domain, using one of the EGFR ligand binding domains as starting template. Previous work has shown the human EGFR domain 3 (herein referred to as d3-wt) to be the ectodomain encoding most of the binding information to the EGF ligand. Therefore, the inventors have used d3-wt as a starting template, and combinatorial design was performed to stabilize the EGF-bound conformation. The d3-wt sequence (Table 1) was restricted to a stretch of 168 amino acids, which contains disulfide bridges at the beginning and the end of the domain. MD simulations have shown these to act strong tethers stabilizing the overall structure. Since the inventors deliberately aimed the designs not to include any cysteine residues, they truncated the domain boundary at the C-terminus to only include 160 amino acids. The designable positions were set to comprise all of the high energy residues as identified by the Damietta energy function (to be above 20 kcal/mol), which were identified using the repack all (ra) protocol. Additionally, all of the of original disulfide bridges positions were forcibly designed; removing all cysteines from the resulting designs. Running 100 instances of the tree swarm combinatorial sampler (cs_f2m2f) with randomized order of the designable positions yielded about 200 decoys with unique sequences. These were subject to accelerated molecular dynamics (AMD) simulations, and were ranked according to their conformational stability. The conformational stability scores as well as the RMSF plots derived from the latter simulations (Fig. 1) indicated a similar stability of the top 10 designs to d3-wt in case of well-formed disulfide bridges, and a better stability of the designs with d3-wt models where the cysteins were reduced. The two most mutually distant sequences in the top 10 designs were eventually selected for experimental evaluation, named dd3-1, and dd3-2 (Fig. 2A, and Methods section).

**Table 1: Protein sequences of template and designed inhibitors proteins.**

| Name | Sequence |
|---|---|
| d3-wt | CNGIGIGEFKDSLSINATNIKHFKNCTSISGDLHILPVAFRGDSFTHTPPLDPQEL DILKTVKEITGFLLIQAWPENRTDLHAFENLEIIRGRTKQHGQFSLAVVSLNITSL |
| | GLRSLKEISDGDVIISGNKNLCYANTINWKKLFGTSGQKTKIISNRGENSCKATG Q (SEQ ID NO: 4) |
| dd3-1 | ANGIGIGEFKDSLSIWAWNIKHFKNARSISGDLHILPVAFRGDSFTHTPPLEPKE LEILKTVKEITGYLLVQAWPENRTDLHAFENLEIIRGRTKQHGQFSLAVVSLNVT SLGLRSLKEISDGDVIMSGNKNLKMANEMNWKKMFGTSGQKTKIISNRGE (SEQ ID NO: 3) |
| dd3-2 | ANGIGIGEFKDSLSINAWNIKHFKNAQSISGDLHILPVAFRGDSFTHMPPLEPKE LEILKTVKEITGYLLIQAWPENRTDLHAFENLEIIRGRTKQHGQFSLAVVSLNVTS LGLRSLKEISDGDVIMSGNKNLKWANKWNWKKVFGTSGQKTKIISNRGE (SEQ ID NO: 2) |

The designs showed similar thermostability to d3-wt, as evaluated by nanoscale differential scanning fluorimetry (nanoDSF) (Fig. 3). However, the designs showed much stronger EGF inhibition activity in proliferation assays using the EGF-dependent epidermoid carcinoma cell line, A431 (Fig. 2B). Particularly, dd3-2 was the most active design, the proliferation inhibition IC50 was more than 1000-fold lower compared to d3-wt (*IC*_{*50*,*dd3-2*} = 0.32 nM vs. *IC_{50,d3-wt}* = 476 nM). The inhibitory concentration of dd3-2 was only 3-fold higher than cetuximab; a therapeutic anti-EGFR antibody (Fig. 2B). To further evaluate the difference in binding affinities towards EGF, the inventors carried their surface plasmon resonance (SPR) titrations of their binders against immobilized EGF. The results showed that dd3-1, and dd3-2 bind EGF 5- and 7-fold tighter than d3-wt, where dissociation constants (Kd) were 10 nM, 7 nM, and 50 nM for dd3-1, dd3-2, and d3-wt, respectively (Fig. 8A, 9). This may indicate that the stabilization of the bound form of the d3 did result in tighter binding designs. Compared to previous results that weaponised an Fc-chimera of the entire EGFR extracellular segment (-95 kDa per subunit), the dd3-2 design is far smaller (18 kDa), leading to a better protein efficiency (i.e.Δ*Gbind*/*MW*) of the latter (-2.6 kJ/kDa) versus the former (-0.5 kJ/kDa). To further evaluate the capacity of the inventors' designs to bind other related EGFR ligands, the inventors performed SPR binding experiments against HB-EGF and TGF-α, which are also important therapeutic targets for treatment of EGFR-dependent cancers. These results also showed dd3-2 to bind HB-EGF 11-fold tighter than d3-wt, where Kd values of 23 nM and 263 nM were observed for dd3-2 and d3-wt, respectively, and demonstrated dd3-2 to bind TGF-α 3-fold tighter than d3-wt, with Kd values of 805 nM and 221 nM, respectively (Fig. 4B, 5C). This multi-specificity of d3 proteins could be an explanation of their high inhibitory activity against A431 cell line, since cancerous cells are usually characterized by interplay between different autocrine and paracrine EGFR ligands. While the SPR results indicated improved binding affinities of the designs towards different EGFR ligands, the much improved inhibitory activity of the designs in cells can be attributed to the fact that d3-wt has a larger hydrophobic surface and higher aggregation propensity in solution. This difference can be more pronounced at 37 °C over 72 hours in cell-based experiment, which is corroborated by the higher tendency of d3-wt protein to aggregate was also observed during size-exclusion purification (Fig. 6).

To investigate potential effect of the designed inhibitors *in vivo,* the inventors injected equal volume of PBS solution containing cetuximab (positive control), d3-wt or dd3-2 in zebrafish embryos. As negative control, pure PBS was injected. Inhibitors were administered at 4-6 hours post fertilization during 4 days and as a first step survival of embryos was determined every day from 1 dpf to 4 dpf. While almost no effect on survival was observed by any time point following injection of PBS, the highest concentration of dd3-2 (1 mg/ml) was found to have most lethality (48 %) and it led to 20 % mortality at 0.2 mg/ml concentration. While d3-wt caused 10 % lethality at 0.3 mg/ml concentration, which increased to 23 % at 1.3 mg/ml concentration. The inventors also determined the morphological defects present in the surviving embryos at 4 dpf in order to check whether the binders exert specific effects on the EGFR signaling or toxic off-target effects. It has been previously shown that EGFR inhibitors cause developmental defects in head cartilage. To investigate the defects in skeletal development associated with designed inhibitors, the analyzed head cartilage formation at 4 dpf by Alcian blue staining (Fig. 7). In comparison to wild type embryos with completely formed cartilaginous elements of pharyngeal skeleton (Fig. 7A), the embryos with cartilaginous defects or even without any cartilage formation were classified as malformed group (Fig. 7B). Next the resulting cartilage profiles were calculated in four groups (Fig. 7C). The results indicate both d3-wt, and dd3-2 to affect skeletal development in a manner typical for EGFR signaling impairment. In line with above-described biophysical and in cell experiments, dd3-2 caused stronger effect in zebrafish embryos compared to d3-wt.

### 4. Conclusion

The inventors have created non-antibody binder targeting EGF, as well as related EGFR ligands, that is as small as 18 kDa, devoid of any post-translational modifications, readily expressed and purified. To date, this represents the first report of a single-domain EGFR-mimic to inhibit its family of soluble ligands with broad specificity.

### Sequences

**Table 2: Amino acid sequences**

| SEQ ID NO. | Sequence | Designation |
|---|---|---|
| 1 | ANGIGIGEFKDSLSIX1AWNIKHFKNAX2SISGDLHILPVAFRGDSFTHX3PPLEPKELEILKTVKEITGYLLX4QAWPENRTDLHAFENLEIIRGRTKQHGQFSLAVVSLNVT SLGLRSLKEISDGDVIMSGNKNLKX5ANX6X7NWKKX8FGTSGQKTKIISNRGE (SEQ ID NO: 1), | Consensus amino acid sequence of inhibitor protein |
| | wherein | |
| | X₁ = W or N | |
| | X₂ = R or Q | |
| | X₃ = T or M | |
| | X₄ = V or I | |
| | X₅ = M or W | |
| | X₆ = E or K | |
| | X₇ = M or W | |
| | X₈ = M or V. | |
| 2 | ANGIGIGEFKDSLSINAWNIKHFKNAQSISGDLHILPVAFRGDSFTHMPPLEPKELEILKTVKEITGYLLIQAWPENRTDLHAFENLEIIRGRTKQHGQFSLAVVSLNVTSL GLRSLKEISDGDVIMSGNKNLKWANKWNWKKVFGTSGQKTKIISNRGE | Amino acid sequence of dd3-2 |
| 3 | ANGIGIGEFKDSLSIWAWNIKHFKNARSISGDLHILPVAFRGDSFTHTPPLEPKELEILKTVKEITGYLLVQAWPENRTDLHAFENLEIIRGRTKQHGQFSLAVVSLNVTSL GLRSLKEISDGDVIMSGNKNLKMANEMNWKKMFGTSGQKTKIISNRGE | Amino acid sequence of dd3-1 |
| 4 | CNGIGIGEFKDSLSINATNIKHFKNCTSISGDLHILPVAFRGDSFTHTPPLDPQELDILKTVKEITGFLLIQAWPENRTDLHAFENLEIIRGRTKQHGQFSLAVVSLNITSLGL RSLKEISDGDVIISGNKNLCYANTINWKKLFGTSGQKTKIISNRGENSCKATGQ | Amino acid sequence of d3-wt |

## Claims

1. An inhibitor protein of ligands of epidermal growth factor receptor (EGFR), or a pharmaceutically acceptable salt or solvate thereof, wherein the inhibitor protein comprises the amino acid sequence of SEQ ID NO: 1 and variant sequences thereof which are at least 88% homologous to SEQ ID NO: 1, and wherein said inhibitor protein comprising a variant sequence binds to ligands of EGFR, wherein said inhibitor protein is not a full-length polypeptide.

2. The inhibitor protein of claim 1, comprising the amino acid sequence 
ANGIGIGEFKDSLSIX1AWNIKHFKNAX2SISGDLHILPVAFRGDSFTHX3PPLEPKEL
EILKTVKEITGYLLX4QAWPENRTDLHAFENLEIIRGRTKQHGQFSLAVVSLNVTSL
GLRSLKEISDGDVIMSGNKNLKX5ANX6X7NWKKX8FGTSGQKTKIISNRGE (SEQ
ID NO: 1),
 wherein
X₁ = W or N
X₂ = R or Q
X₃ = T or M
X₄ = V or I
X₅ = M or W
X₆ = E or K
X₇ = M or W
X₈ = M or V.

3. The inhibitor protein of claim 1 or 2, comprising the amino acid sequence of SEQ ID NO: 2.

4. The inhibitor protein of claim 1 or 2, comprising the amino acid sequence of SEQ ID NO: 3.

5. An antibody, in particular a soluble or membrane-bound antibody, preferably a monoclonal antibody, or fragment thereof, that specifically recognizes the inhibitor protein according to any of claims 1 to 4.

6. A polynucleotide comprising a nucleotide sequence encoding the inhibitor protein of any of claims 1-4 and/or the antibody or fragment thereof of claim 5, optionally linked to a promoter sequence.

7. A vector comprising the polynucleotide of claim 6.

8. A recombinant host cell comprising the inhibitor protein of any of claims 1-4, and/or the polynucleotide of claim 6, and/or the vector of claim 7.

9. The recombinant host cell of claim 8, which is a bacterial cell, preferably an *Escherichia coli cell.*

10. A pharmaceutical composition comprising the inhibitor protein of any of claims 1-4, and/or the antibody or fragment thereof of claim 5, and/or the polynucleotide of claim 6, and/or the vector of claim 7, and/or the recombinant host cell of claim 8 or 9.

11. The inhibitor protein of any of claims 1-4, and/or the antibody or fragment thereof of claim 5, and/or the polynucleotide of claim 6, and/or the vector of claim 7, and/or the recombinant host cell of claim 8 or 9 for use in medicine, preferably said use is in diagnosis and/or prevention and/or treatment of an EGFR-associated disease, preferably cancer, or for use in the manufacture of a medicament against an EGFR-associated disease, preferably cancer, further preferably said cancer is selected from the group consisting of: colon cancer, epidermoid cancer, adenocarcinoma, anal cancer, glioblastoma and epithelian cancer.

12. A kit comprising:
(a) a container comprising the inhibitor protein of any one of claims 1-4, and/or the antibody or fragment thereof of claim 5, and/or the polynucleotide of claim 6, and/or the vector of claim 7, and/or the recombinant host cell of claim 8 or 9, in solution or in lyophilized formulation;
(b) optionally, a second container containing a diluent or reconstituting solution for the lyophilized formulation;
(c) optionally, instructions for (i) use of the solution or (ii) reconstitution and/or use of the lyophilized formulation.

13. A method of treating an EGFR-associated disease in a subject in need, comprising administering to the subject an effective amount of the inhibitor protein of any one of claims 1-4, and/or the antibody or fragment thereof of claim 5, and/or the polynucleotide of claim 6, and/or the vector of claim 7, and/or the recombinant host cell of claim 8 or 9, or the pharmaceutical composition of claim 10.

14. The method of claim 13, wherein the EGFR-associated disease is cancer, preferably said cancer is selected from the group consisting of: colon cancer, epidermoid cancer, adenocarcinoma, anal cancer, glioblastoma and epithelian cancer.

15. The method of claim 13 or 14, wherein the inhibitor protein, and/or the polynucleotide, and/or the vector, and/or the recombinant host cell, and/or the pharmaceutical composition are administered to the subject by an oral, parenteral, intravenous, peritoneal, intradermal, subcutaneous, intramuscular, intrathecal, inhalation, vaporization, nebulization, sublingual, buccal, parenteral, rectal, intraocular, inhalation, topically, vaginal, or topical route of administration.
